# EUROPÄISCHE PATENTSCHRIFT

(11) **EP 2 532 323 B1**
(45) Veröffentlichungstag und Bekanntmachung des Hinweises auf die Patenterteilung: **07.02.2018**
(21) Anmeldenummer: 12004236.1
(22) Anmeldetag: 04.06.2012
(51) Int. Cl.: A61F 2/38, A61F 2/30

(54) **Form zur Herstellung einer Tibiakomponente eines Kniegelenkspacers**
Mould for producing a tibia component of a knee joint spacer
Moule destiné à la fabrication d'un composant de tibia d'un espaceur d'articulation du genou

(30) Priorität: 06.06.2011 DE 102011104808
(43) Veröffentlichungstag der Anmeldung: 12.12.2012
(73) Patentinhaber: Heraeus Medical GmbH, 61273 Wehrheim (DE)
(72) Erfinder: Wüst, Edgar, 64823 Groß-Umstadt (DE)
(74) Vertreter: Heraeus IP

(56) Entgegenhaltungen:
- EP-B1- 1 082 199
- US-A- 5 123 927
- US-A1- 2010 102 484

## Beschreibung

Die vorliegende Erfindung betrifft eine Form zur Herstellung einer Tibiakomponente eines Kniegelenkspacers, sie betrifft insbesondere eine mehrteilige Form.

In Deutschland werden zurzeit ca. 150.000 Knietotalendoprothesen pro Jahr implantiert. Als relevante Komplikationen können Früh- und Spätinfektionen des Implantatlagers auftreten. Kommt es zur Infektion im Bereich des künstlichen Gelenkes, wird zunächst versucht, den Infekt mittels konsequenter chirurgischer Sanierung kombiniert mit testgerechten Antibiotika zu behandeln. In sehr vielen Fällen ist dies alleine jedoch nicht wirksam. Die infizierte Prothese muss dann operativ entfernt werden. Dabei wird gleichzeitig der durch die Infektion kompromittierte Knochen mit entfernt.

Nachdem die infizierte Prothese entfernt wurde, platziert man, um die Stabilität des Gelenkes aufrecht zu erhalten, an Stelle der Prothese einen sogenannten Platzhalter oder Spacer.

Die zurzeit auf dem Markt befindlichen Spacer bestehen aus Kunststoff (PMMA). Die Geometrie der Kniegelenkspacer orientiert sich im Groben am Design einer Primärprothese. Unberücksichtigt bleiben dabei die individuellen Zuschnitte am Knochen, die von der ursprünglich implantierten Prothese vorgegeben sind. Die bisherigen verfügbaren Spacer können nicht wieder aufbereitet werden, das heißt sie dienen zum Einmalgebrauch.

Alternativ zu den oben genannten Spacern verwenden manche Operateure Platzhalter, die während der Operation eigenhändig aus Knochenzement hergestellt werden, die wahlweise antibiotisch wirksame Bestandteile enthalten können. Dabei handelt es sich um Provisorien, zu deren Herstellung Formen verwendet werden.

Spacer oder Platzhaltervorrichtungen für das Kniegelenk weisen einen ZweiKomponenten-Aufbau auf, wobei eine Femurkomponente (verbunden mit dem Oberschenkelknochen) und eine Tibiakomponente (verbunden mit dem Schienbeinplateau) zu einem derartigen Kniegelenkspacer gehören.

So wird zum Beispiel in EP 1 082 199 B1 eine Zementform für ein provisorisches Implantat beschrieben, bei dem es sich unter anderem auch um die Tibiakomponente eines Kniegelenkspacers handelt. Die Form besteht aus zwei Teilformen. Bei der ersten Teilform handelt es sich um eine Schale, die aus einem im Wesentlichen planen Boden und einer umlaufenden Wand besteht. Die Form besteht aus einem flexiblen Silikonmaterial und wird, nachdem der Zementteig eingefüllt und ausgehärtet ist, auseinandergebogen, um das provisorischen Implantat aus der Form entnehmen zu können.

Ein Nachteil dieser Form besteht darin, dass sie elastisch ist, und demzufolge der Spacer nicht immer exakt ausgeformt wird. Ein Nacharbeiten ist häufig notwendig.

In US 2010/0102484 A1 ist ferner eine Tibia-Form beschrieben, die mindestens zwei Formteile aufweist, wobei die Formteile mit Hilfe von Befestigungseinrichtungen über einen Stift in unterschiedlichen Positionen zueinander angeordnet werden können.

Eine Aufgabe der vorliegenden Erfindung besteht daher darin, eine Form für die Tibiakomponente eines Kniegelenkspacers bereitzustellen, mit deren Hilfe Tibiakomponenten hergestellt werden können, die gar nicht oder nur geringfügig nachgearbeitet werden müssen.

Diese Aufgabe wird durch den Gegenstand mit den Merkmalen des Anspruchs 1 gelöst. Weiterbildungen der Erfindung sind durch die Unteransprüche definiert.

Erfindungsgemäß weist die Form für die Tibiakomponente eines Kniegelenkspacers eine Hülse zur Ausbildung des Randbereichs der Tibiakomponente, und einen verschiebbar in der Hülse angeordneten Stempel zur Ausbildung der femurseitigen Oberfläche der Tibiakomponente auf, wobei die Form ferner einen Deckel zur Ausbildung der tibiaseitigen Oberfläche der Tibiakomponente, und Fixiereinrichtungen an der Hülse und am Stempel aufweisen, um den Stempel in unterschiedlichen Positionen in der Hülse zu fixieren, wobei die Form ferner einen Deckel zur Ausbildung der tibiaseitigen Oberfläche der Tibiakomponente aufweist, und die Fixiereinrichtungen vom Außenrand ausgehende senkrechte Nuten sind, in die am Stempel ausgebildete vorstehende Elemente eingeschoben werden können, derart, dass der Stempel kontinuierlich verschiebbar ist und die Dicke der Tibiakomponente einstellbar ist.
Zur Herstellung der Tibiakomponente eines Kniegelenkspacers wird zunächst ein Knochenzementteig hergestellt. Patientenspezifisch kann gleichzeitig ein oder mehrere entsprechende Antibiotika unter den Teig gerührt werden. Dieser Knochenzementteig wird in die Form eingefüllt, solange der Deckel geöffnet ist, der Stempel jedoch bereits in die Hülse eingesetzt ist. Er wird bis zum Rand in die Form eingefüllt. Anschließend wird der Deckel aufgesetzt, so dass die Tibiaseite ausgeformt wird. Die Dicke der Tibiakomponente wird durch die Anordnung des Stempels in der Hülse bestimmt.
Die Hülse weist an ihrem Innenumfang Fixiereinrichtungen auf, die zusammen mit Fixiereinrichtungen am Stempel den Stempel in Position halten.
Die Fixiereinrichtungen sind vom Außenrand ausgehende senkrechte Nuten, in die am Stempel ausgebildete vorstehende Elemente eingeschoben werden können. Auf diese Weise kann der Stempel nicht nur in vorgegebenen Höhen in der Hülse fixiert werden. Er kann vielmehr kontinuierlich verschoben werden und damit die Dicke der Tibiakomponente eingestellt werden.
Bevorzugt weist der Deckel eine Öffnung auf, durch die überschüssiger Knochenzement herausgedrückt wird. Es ist ebenfalls von Vorteil, wenn in den Deckel Entlüftungslöcher eingelassen sind. Luft, die eventuell noch zwischen Knochenzement und Deckel enthalten ist, kann so entweichen. Es können sich keine Lufträume bilden, die zu Fehlstellen in der Tibiakomponente führen könnten.

Insbesondere in Fällen von Knochenverlust kann es notwendig sein, dass die Tibiakomponente einen Schaft aufweist. Er dient dazu, das Implantat in der Tibia zu stabilisieren und die Belastung in der Tibia zu verteilen. Soll die Tibiakomponente einen Schaft haben, so ist ein weiteres Formteil zur Ausbildung eines Schaftes an dem Deckel angeordnet. Dieses Formteil hat bevorzugt eine längliche Form, die sich ausgehend vom Deckel zu ihrem distalen Ende hin verjüngt. Sie kann an ihrem distalen Ende offen oder geschlossen sein. Um eine Tibiakomponente mit einem Schaft herzustellen, werden zunächst das Formteil für die Tibiawanne und das Formteil für den Schaft gefüllt. Anschließend wird der Deckel samt Schaft auf die Form gesetzt. Die Knochenzemente werden zusammengepresst und bilden nach dem Aushärten zusammen die Tibiakomponente mit Schaft.

Die Formen können aus jedem beliebigen biokompatiblen Material mit ausreichender Festigkeit hergestellt werden. Es gibt verschiedene Polymere, die für diese Anwendungen geeignet sind. Besonders geeignet sind formstabile, harte Materialien. Beispiele hierfür sind Polyethylen, Polypropylen, Polyamid, Polyetherketon, Teflon und Polyoxymethylen. Besonders geeignet sind glaskugelverstärkte thermoplastische Kunststoffe, wie glaskugelverstärktes Polyethylen, glaskugelverstärktes Polypropylen, glaskugelverstärktes Polyamid. Diese Materialien haben eine ausreichende Festigkeit, so dass sie sich während der Herstellung der Spacer nicht verformen.

Prinzipiell sind alle biokompatiblen Materialien geeignet, aus denen sich die Formen herstellen lassen, unter der Voraussetzung, dass sie keine chemische Reaktion mit Knochenzement oder Antibiotikum eingehen. Denkbar sind somit auch Metalle, wie z.B. Edelmetall oder teflonbeschichtetes Aluminium.

Erfindungsgemäß bedeutet "formstabil", dass sich die Form während des Füllvorgangs nicht verzieht, so dass die Tibiakomponente exakt ausgeformt wird und mit der Femurkomponente eine formschlüssige Gleitpaarung bildet, ohne dass ein Nacharbeiten notwendig wird.

Im Folgenden werden bevorzugte Ausführungsformen der vorliegenden Erfindung anhand der beiliegenden Zeichnungen genauer beschrieben. Es zeigen:
Fig. 1 eine perspektivische Darstellung eines Teils einer bevorzugten Ausführungsform einer erfindungsgemäßen Form in auseinandergenommenem Zustand,
Fig. 2 eine weitere perspektivische Darstellung der Form nach Fig. 1 in zusammengesetztem Zustand,
Fig. 3 eine perspektivische Darstellung eines anderen Teils der Form nach Fig. 1,
Fig. 4 einen Teil einer dritten bevorzugten Ausführungsform einer erfindungsgemäßen Form, und
Fig. 5 eine Tibiakomponente nach Entnahme aus einer Form.

In den Figuren bezeichnen dieselben Bezugszeichen gleiche oder funktionsgleiche Komponenten, soweit nichts Gegenteiliges angegeben ist.

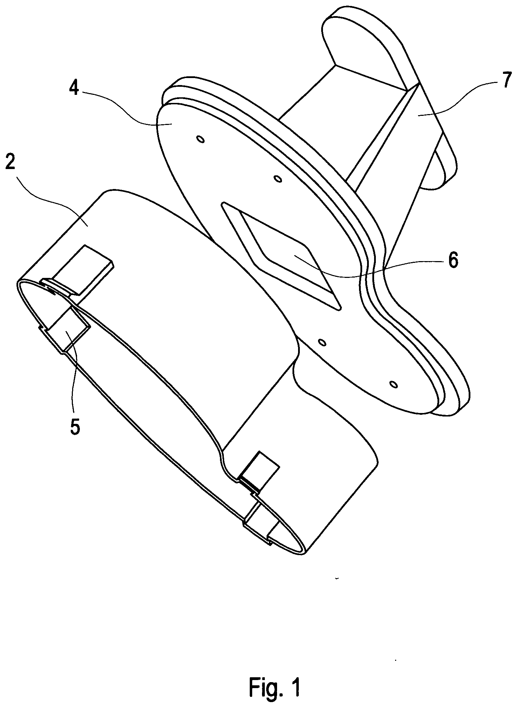

Fig. 1 zeigt eine perspektivische Darstellung eines Ausführungsbeispiels einer erfindungsgemäßen Form 1 zur Herstellung der Tibiakomponente eines Kniegelenkspacers. Die Gestalt (Innenkontur) der Form 1 richtet sich nach dem Implantat, welches die Tibiakomponente für eine gewisse Zeit ersetzen soll. Die Form 1 weist mehrere Elemente auf. Für die Ausbildung des Umfangs der Tibiakomponente dient eine Hülse 2, die auf einer Seite durch einen Deckel 4 abgedeckt ist. Auf der anderen Seite der Hülse 2 sind Fixiereinrichtungen 5 vorgesehen, in denen ein Stempel in verschiedenen Höhen in der Hülse fixiert werden kann. Der Deckel 4 weist eine Öffnung 6 auf, durch die überschüssiger Zementteig gedrückt wird.

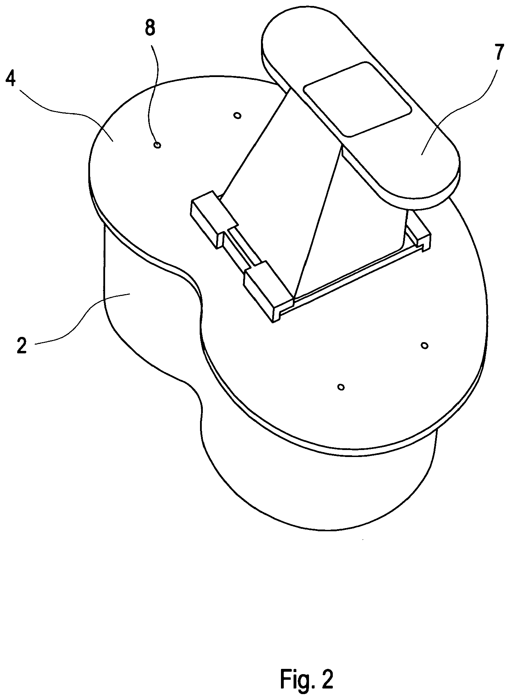

Fig. 2 zeigt die gleiche Form von einer anderen Seite. Die Form ist mit dem Deckel 4 abgedeckt, der mittig eine Öffnung aufweist. Ausgehend von der Öffnung erstreckt sich senkrecht zum Deckel 4 ein Formteil 7 zur Ausbildung eines Schaftes. Die innere Querschnittsfläche des Formteils 7 für den Schaft verjüngt sich von der Tibiakomponente ausgehend zu ihrem distalen Ende.

Es ist von Vorteil, wenn der Deckel 4 Löcher aufweist, durch die Luft entweichen kann. Soll die Tibiakomponente einen Schaft haben, so wird zunächst die Form 7 zur Ausbildung des Schaftes gefüllt und erst dann der Deckel 4 aufgesetzt. Die Knochenzementmasse der Tibiakomponente und die Knochenzementmasse des Schaftes verbinden sich beim Aushärten dann zu einem Formteil.

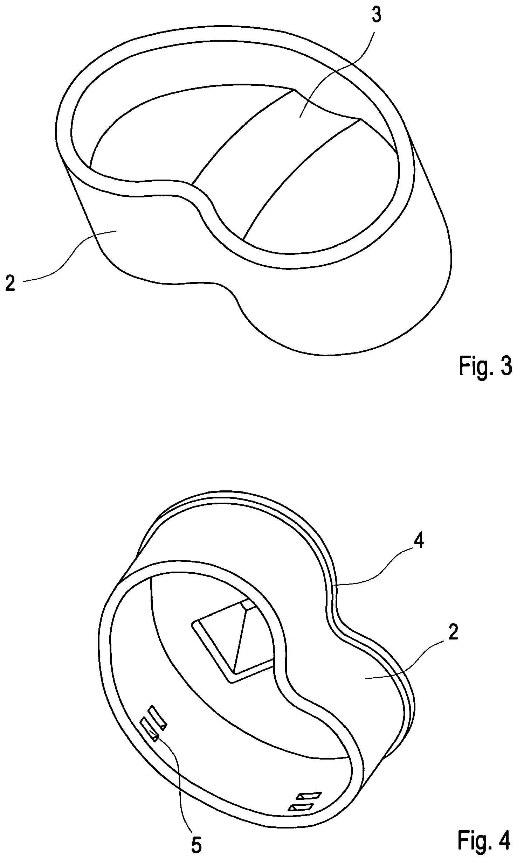

Fig. 3 zeigt einen Ausschnitt der Form 1, bei der der Deckel weggelassen wurde. In der Form ist ein Stempel 3 angeordnet. Durch die in Fig. 1 gezeigten Fixiereinrichtungen kann der Stempel 3 in unterschiedlichen Positionen in der Hülse 2 fixiert werden. Auf diese Weise kann die Dicke der Tibiakomponente angepasst werden.

Fig. 4 zeigt eine weitere Ausführungsform der Fixiereinrichtungen 5. In diesem Fall handelt es sich um senkrecht in die Hülse eingelassene Schlitze, die eine Art Schiene für dazu passende Einrichtungen bilden, welche am Stempel ausgebildet sind. In diesem Fall kann der Stempel kontinuierlich über die Länge der Schiene verschoben werden.

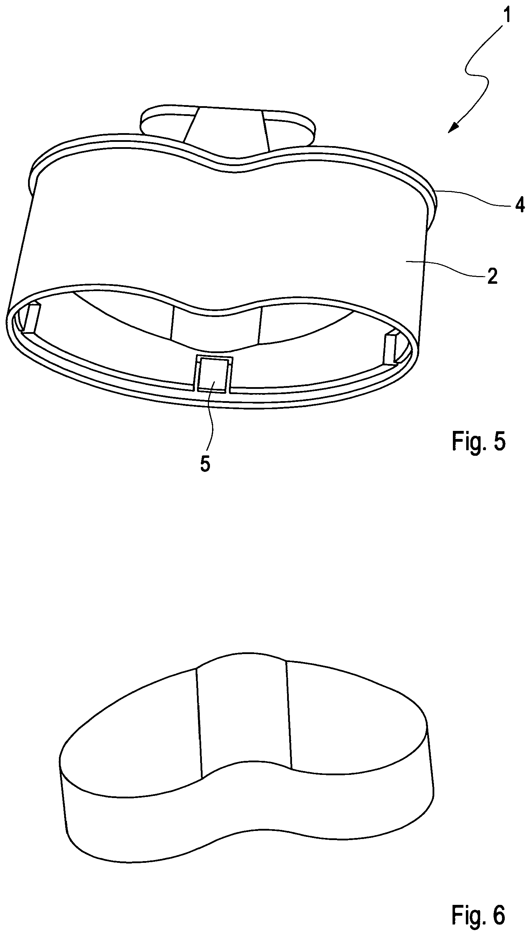

Fig. 5 zeigt die Tibiakomponente eines Kniegelekspacers nach Entnahme aus der Form.

### Bezugszeichenliste

- 1: Form
- 2: Hülse
- 3: Stempel
- 4: Deckel
- 5: Fixiereinrichtungen
- 6: Öffnung
- 7: Formteil für einen Schaft
- 8: Entlüftungslöcher

## Patentansprüche

1. Form zur Herstellung der Tibiakomponente eines Kniegelenkspacers, aufweisend eine Hülse (2) zur Ausbildung des Randbereichs der Tibiakomponente und einen Deckel (4) zur Ausbildung der tibiaseitigen Oberfläche der Tibiakomponente, **dadurch gekennzeichnet, dass** die Form ferner
einen verschiebbar in der Hülse (2) angeordneten Stempel (3) aufweist, der geeignet ist zur Ausbildung der femurseitigen Oberfläche der Tibiakomponente, wobei
die Form Fixiereinrichtungen (5) an der Hülse (2) und am Stempel (3) aufweist, um den Stempel (3) in unterschiedlichen Positionen in der Hülse (2) zu fixieren,
und die Fixiereinrichtungen vom Außenrand ausgehende senkrechte Nuten sind, in die am Stempel ausgebildete vorstehende Elemente eingeschoben werden können, derart, dass der Stempel kontinuierlich verschiebbar ist und die Dicke der Tibiakomponente einstellbar ist.

2. Form nach einem der vorhergehenden Ansprüche,
**dadurch gekennzeichnet, dass**
der Deckel (4) eine Öffnung (6) aufweist.

3. Form nach Anspruch 2,
**dadurch gekennzeichnet, dass**
die Form außerdem ein Formteil (7) zur Ausbildung eines Tibiaschaftes aufweist, welches die Öffnung (6) umgibt und dessen innere Querschnittsfläche sich von der Öffnung (6) ausgehend verjüngt.

4. Form nach einem der vorhergehenden Ansprüche,
**dadurch gekennzeichnet, dass**
die Form (1) aus einem formstabilen Material gefertigt ist.

5. Form nach einem der vorhergehenden Ansprüche,
**dadurch gekennzeichnet, dass**
es sich bei dem Material um einen Kunststoff handelt.

6. Form nach Anspruch 5,
**dadurch gekennzeichnet, dass**
der Kunststoff aus der Gruppe ausgewählt ist, die aus Polypropylen, Polyethylen und Polyamid besteht.

7. Form nach Anspruch 6,
**dadurch gekennzeichnet, dass**
der Kunststoff ein glaskugelverstärktes Polypropylen oder Polyamid ist.

## Claims

1. Mould for the production of the tibial component of a knee joint spacer, comprising a sleeve (2) for forming the marginal region of the tibial component and a cover (4) for forming the tibia-side surface of the tibial component, **characterised in that** the mould further comprises a punch (3) that is arranged appropriately such that it can be shifted in the sleeve (2) and is suitable for forming the femur-side surface of the tibial component, whereby the mould comprises fixing facilities (5) on the sleeve (2) and on the punch (3) in order to affix the punch (3) in different positions in the sleeve (2), and the fixing facilities are perpendicular grooves originating from the outer margin into which projecting elements, which are formed on the punch, can be inserted such that the punch can be shifted continuously and the thickness of the tibial component can be adjusted.

2. Mould according to any one of the preceding claims, **characterised in that** the cover (4) comprises an opening (6).

3. Mould according to claim 2, **characterised in that** the mould further comprises a mould part (7) for forming a tibial stem, which surrounds the opening (6) and whose inner cross-sectional surface tapers originating from the opening (6).

4. Mould according to any one of the preceding claims, **characterised in that** the mould (1) is manufactured from a shape-stable material.

5. Mould according to any one of the preceding claims, **characterised in that** the material is a plastic material.

6. Mould according to claim 5, **characterised in that** the plastic material is selected from the group consisting of polypropylene, polyethylene, and polyamide.

7. Mould according to claim 6, **characterised in that** the plastic material is a glass bead-reinforced polypropylene or polyamide.

## Revendications

1. Moule pour la fabrication du composant tibial d'un écarteur d'articulation du genou, présentant un manchon (2) pour la réalisation de la région de bord du composant tibial et un couvercle (4) pour la réalisation de la surface du côté tibial du composant tibial, **caractérisé en ce que** le moule présente en outre un poinçon (3) disposé de manière à pouvoir être coulissé dans le manchon (2) qui convient à la réalisation de la surface du côté fémoral du composant tibial, dans lequel le moule présente des dispositifs de fixation (5) sur le manchon (2) et sur le poinçon (3) pour fixer le poinçon (3) dans différentes positions dans le manchon (2), et les dispositifs de fixation sont des rainures verticales partant du bord externe dans lesquelles des éléments saillants réalisés sur le poinçon peuvent être insérés de telle sorte que le poinçon peut être coulissé en continu et l'épaisseur du composant tibial peut être réglée.

2. Moule selon une des revendications précédentes, **caractérisé en ce que** le couvercle (4) présente une ouverture (6).

3. Moule selon la revendication 2, **caractérisé en ce que** le moule présente en outre une partie de moule (7) pour la réalisation d'une diaphyse tibiale qui entoure l'ouverture (6) et dont la surface transversale interne se rétrécit en partant de l'ouverture (6).

4. Moule selon une des revendications précédentes, **caractérisé en ce que** le moule (1) est fabriqué en un matériau indéformable.

5. Moule selon une des revendications précédentes, **caractérisé en ce qu'**il s'agit pour le matériau d'un plastique.

6. Moule selon la revendication 5, **caractérisé en ce que** le plastique est sélectionné parmi le groupe qui se compose du polypropylène, polyéthylène et polyamide.

7. Moule selon la revendication 6, **caractérisé en ce que** le plastique est un polypropylène ou polyamide renforcé par des billes de verre.
